(19) Europäisches Patentamt
European Patent Office
Office européen des brevets

(11) Veröffentlichungsnummer: **0 080 977**
**B1**

(12) # EUROPÄISCHE PATENTSCHRIFT

(45) Veröffentlichungstag der Patentschrift: **05.11.86**

(51) Int. Cl.⁴: **A 61 K 7/06**

(21) Anmeldenummer: **82810504.9**

(22) Anmeldetag: **24.11.82**

(54) **Gemische aus quaternären, polymeren Ammoniumsalzen, aus quaternären, mono- bis oligomeren Ammoniumsalzen und aus alkoxylierten oder sulfonierten Tensiden, deren Herstellung und Verwendung in kosmetischen Mitteln.**

(30) Priorität: **30.11.81 CH 7656/81**

(43) Veröffentlichungstag der Anmeldung: **08.06.83 Patentblatt 83/23**

(45) Bekanntmachung des Hinweises auf die Patenterteilung: **05.11.86 Patentblatt 86/45**

(84) Benannte Vertragsstaaten: **CH DE FR GB IT LI**

(56) Entgegenhaltungen:
**EP-A-0 047 714**
**FR-A-2 113 845**
**FR-A-2 252 840**
**FR-A-2 312 232**
**FR-A-2 327 761**
**FR-A-2 355 497**
**GB-A-2 006 237**
**GB-A-2 050 166**
**GB-A-2 065 472**
**GB-A-2 066 663**
**US-A-4 269 824**

**PATENTS ABSTRACTS OF JAPAN, Band 3, Nr. 68, 13. Juni 1979, Seite 128C48, Tokyo, JP**
**CHEMICAL ABSTRACTS, Band 92, Nr. 14, April 1980, Seite 343, Nr. 116256s, Columbus, Ohio, USA**
**PATENTS ABSTRACTS OF JAPAN, Band 5, Nr. 75, 19. Mai 1981, Seite 747C55, Tokyo, JP**

(73) Patentinhaber: **CIBA- GEIGY AG, Klybeckstrasse 141, CH- 4002 Basel (CH)**

(72) Erfinder: **Moldovanyi, Laszlo, Dr., Austrasse 78, CH- 4051 Basel (CH)**
Erfinder: **Fearnley, Charles Dr., Wettsteinanlage 50, CH- 4125 Riehen (CH)**
Erfinder: **Hungerbühler, Walter, Höhenstrasse 8, CH- 4125 Riehen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

EP 0 080 977 B1

**Beschreibung**

In der Haarpflege stellt die Reinigung der Haare einen der wichtigsten Vorgänge dar. Neuerdings werden als Reinigungsmittel zweckmässig synthetische Waschrohstoffe eingesetzt, die den Vorteil aufweisen, auch bei hoher Wasserhärte ihre volle Reinigungskraft zu behalten. Bei alleiniger Verwendung von Waschrohstoffen zeigt das behandelte Haar in der Regel keine Konditioniereffekte.

Der vorliegenden Erfindung liegt nun die Aufgabe zugrunde, ein für kosmetische Zwecke, insbesondere für die Haarpflege geeignetes kostengünstiges Reinigungsmittel zur Verfügung zu stellen, das neben einer guten Waschwirkung die Erzielung ausgezeichneter Konditioniereffekte auf dem Haar ermöglicht. Diese sog. Konditioniereffekte stellen einen Sammelbegriff für u.a. folgende erwünschte Eigenschaften des behandelten Haares dar:

- leichte Nass- und Trockenkämmbarkeit
- Verhinderung der statischen Aufladung
- keine negative Beeinträchtigung des ursprünglichen Glanzes
- Aspekt, insbesondere Volumen und Fülle
- Griff.

Gute Konditioniereffekte sind mit monomeren, quaternären Ammoniumsalzen insbesomdere in Nachspülmitteln erreichbar. Die Affinität dieser Ammoniumsalze zum Haarkeratin ist jedoch im allgemeinen nur in Abwesenheit von synthetischen Waschrohstoffen, insbesondere von anionischen Tensiden, voll gewährleistet, so dass der Einsatz solcher Ammoniumsalze in Shampoos kaum in Frage kommt. Um an sich gute Konditioniereffekte zu erzielen, ist es deshalb in der Regel nötig, das Haar zuerst zu shampoonieren und in einer getrennten, weiteren Stufe mit einem tensidarmen Nachspülmittel zu behandeln, das das angegebene quaternäre Ammoniumsalz enthält. Obwohl die monomeren, quaternären Ammoniumsalze an sich im allgemeinen besonders preisgünstig sind, ist ihre begrenzte Formulierungsmöglichkeit ein wesentlicher Nachteil, da immer ein zweistufiges und damit recht aufwendiges Haarbehandlungsverfahren erforderlich ist.

Auch mit hochmolekularen, polymeren, quaternären Ammoniumsalzen sind gute Konditioniereffekte erreichbar, die jedoch im allgemeinen schwächer sind als beim gleichzeitigen Einsatz von monomeren, quaternären Ammoniumsalzen. Dies trifft vor allem für die Verhinderung der statischen Aufladung der behandelten Haare zu. Obschon die polymeren, quaternären Ammoniumsalze in Gegenwart von vielen, in die Kosmetikindustrie üblicherweise verwendeten Tensiden ihre Affinität zum Haarkeratin behalten und somit in den meisten Shampooformulierungen einsetzbar sind, weisen sie neben ihrem relativ hohen Preis den wesentlichen Nachteil auf, zusammen mit gewissen Tensiden bei der Haarbehandlung zu unerwünschten Akkumuliereffekten führen zu können.

Ferner können viele Tenside nicht in Kombination mit (A) hochmolekularen, polymeren und (B) monomeren, quaternären Ammoniumsalzen eingesetzt werden, da keine stabilen Formulierungen erhalten werden.

Andererseits benötigt man zur Erreichung von guten Konditioniereffekten in der Regel nur teure Tensidgemische, während kostengünstige Tenside, die normalerweise als Einzelverbindungen vorliegen, nur zu schwachen Konditioniereffekten führen.

Durch die Kombination von ausgewählten, monomeren, quaternären Ammoniumsalzen mit ausgewählten, polymeren, quaternären Ammoniumsalzen in Gegenwart von ausgewählten Tensiden in neuartiger Zusammensetzung ist es erfindungsgemäss nun auf unerwartete Weise gelungen, die vorstehenden, erwähnten Nachteile der separaten Verwendung der Ammoniumsalze der beiden angegebenen Arten weitgehend zu eliminieren.

Erfindungsgemäss werden polymere, quaternäre Ammoniumsalze eingesetzt, die quatermierte Stärke-, Cellulose- oder Guargummiderivate oder Copolymerisate aus Dialkenyldialkylammoniumsalzen und (Meth)acrylamid oder aus Meth(acryl)säure, -amid oder -nitril und quaterniertem Ammoniumsalzen der Acrylsäurereihe enthalten. Im eingesetztem, monomeren Ammoniumsalz ist das quaternäre Stickstoffatom mit zwei Methyl-, Aethyl- oder Hydroxyäthylresten, einem $C_{10}$-$C_{22}$-Alkylrest und einem $C_{10}$-$C_{22}$ Alkyl-, Benzyl-, Phenoxymethylen- oder Phenoxyäthylenrest substituiert. Als anionisches Tensid werden oberflächenaktive Sarkosinate, Sulfate, Sulfatäther, Sulfonate oder Sulfosuccinate eingesetzt.

Aus FR-A-2 252 840, FR-A-2 312 232, FR-A-2 327 761, GB-A-2 006 237, GB-A-2 065 472 und GB-A-2 066 663 sind Zusammensetzungen bekannt, die neben monomeren Ammoniumsalzen und anionischen Tensiden polymere Ammoniumsalze enthalten, die jedoch aus vernetzten Polymer aus einem Polyaminopolyamid oder aus einem Graftterpolymeren aus Vinylpyrrolidon, einem quaternären Acrylat und Polyäthylenglykol bestehen oder aus funktionellen Verbindungen mit Hydroxylrestem stets in Gegenwart von Cerium Ionen oder aus ditertiären Diaminen erhalten werden.

Aus US-A-4 269 824 und JP-A-79 135 234 sind Zusammensetzungem bekannt, die neben quaternäre Cellulosederivate aufweisenden, polymerem Ammoniumsalzen und monomerem Ammoniumsalzen jedoch nicht-ionogene Tenside enthalten.

Aus FR-A-2 355 497 und JP-A-5 443 211 sind Zusammensetzungen bekannt, die neben quaternäre Cellulosederivate aufweisenden, polymeren Ammoniumsalzen und anionischen Tensiden monomere Ammoniunsalze enthalten, die jedoch gegebenenfalls mit Kohlenwasserstoffresten mit höchstens 6 Kohlenstoffatomen substituiert sind oder Alkylenoxyd Additionsprodukte darstellen.

Im Gegensatz hierzu besteht der Gegenstand der vorliegenden Erfindung aus wassrigen Zusammensetzungen aus polymeren, quaternären Ammoniumsalzen, aus monomeren, quaternären

Ammoniumsalzen und aus anionischen, sulfonierten Tensiden oder Sarkosinattensiden, die dadurch gekennzeichnet sind, dass sie einen pH-Wert von 3 bis 8 aufweisen und

(A) 0,05 bis 1,5 Gewichtsteile, berechnet als Wirksubstanz, eines in wässrigen Tensidsystemen löslichen oder mikroemulgierbaren, polymeren Ammoniumsalzes mit mindestens 6 quaternären Stickstoffatomen und einem Molekulargewicht von $10^3$ bis $5x10^6$, das quaternierte Stärke-, Cellulose- oder Guargummi-Derivate, Copolymerisate aus Dialkenyldialkylammoniumsalzen und Acrylamid oder Methacrylamid oder Copolymerisate aus Acryl- oder Methacrylsäure, -amid oder -nitril und quaternierten Ammoniumsalzen der Acrylsäurereihe enthält.

(B) 0,2 bis 10 Gewichtsteile, berechnet als Wirksubstanz, eines Ammoniumsalzes der Formel

(1) 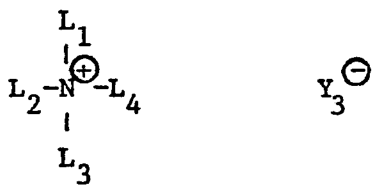

worin $L_1$ und $L_2$ je Methyl, Aethyl oder Hydroxyäthyl, $L_3$ Alkyl mit 10 bis 22 Kohlenstoffatomen, $L_4$ Alkyl mit 10 bis 22 Kohlenstoffatomen, Benzyl, Phenoxymethylen oder Phenoxyäthylen und $Y_3^{\ominus}$ ein Chlorid-, Methylsulfat- oder Acetatanion bedeuten, und

(C) 5 bis 20 Gewichtsteile, berechnet als Wirksubstanz, eines anionischen Tensids der Formel

(2) $$X^{\ominus}Z^{\oplus} ,$$

worin $X^{\ominus}$ den Rest eines oberflächenaktiven Sarkosinates, Sulfats (Sulfatäthers), Sulfonates oder Sulfobernsteinsäurederivates und $Z^{\oplus}$ in Alkalimetall- oder ein gegebenenfalls durch ($C_1$-$C_4$)-Alkyl-oder -Alkanolreste substituiertes Ammoniumkation bedeuten, enthalten,

wobei das anionische Tensid mindestens teilweise mit den Komponenten (A) und (B) unter Ionenaustausch umgesetzt ist, und die Zusammensetzungen mit entionisiertem Wasser auf insgesamt 100 Gewichtsteile verdünnt sind.

Bevorzugte, quaternäre Stärke-, Cellulose- oder Guargummiderivate weisen als Komponente (A) ein Molekulargewicht von $10^3$ bis $10^6$, vorzugsweise $10^4$ bis $10^6$, und Stärke- oder Cellulose-Einheiten oder Polygalaktomannan-Einheiten aus Guargummi auf, die vorzugsweise mit dem 1-Trimethylammomium-2-hydroxypropyl-Rest mindestens teilweise veräthert sind.

Insbesondere weisen die Stärke-, Cellulose- oder Polygalaktomannan-Einheiten solcher Derivate Reste der Formel

(3) 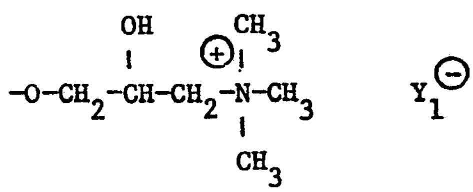

worin $Y_1^{\ominus}$ ein Halogenid-, Alkylsulfat- oder Alkylphosphonatanion oder das Anion einer Alkylcarbonsäure mit je 1 bis 4 Kohlenstoffatomen im Alkylrest bedeutet, und gegebenenfalls auch Reste der Formeln

(4) $$-O-(CH_2-CH_2-O)_{m_1}-H ,$$

(5) 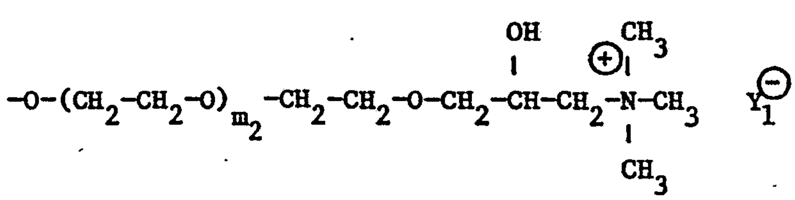

und

(6) 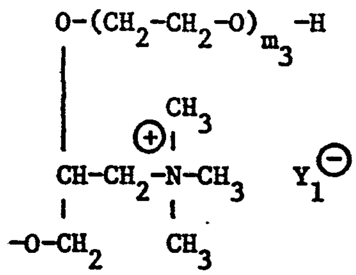

auf,

worin $m_1$, $m_2$ und $m_3$ jeweils ganze Zahlen von 1 bis 50 bedeuten und $Y_1^{\ominus}$ die angegebenen Bedeutungen hat.

Cellulose-Derivate dieser Art sind z.B. in FR A-1 492 597 beschrieben und quaternäre Guargummi-Derivate dieser Art sind z.B. in DE-A-3 018 600 genannt.

Bevorzugte Copolymerisate aus Dialkenyldialkylammoniumsalzen und Acrylamid oder Methacrylamid als Komponente (A) sind z.B. Copolymerisate aus Dimethyldiallylammoniumchlorid und Acrylamid, die ein Molekulargewicht von $5x10^3$ bis $5x10^6$, vorzugsweise $10^4$ bis $3x10^6$ und in beliebiger Reihenfolge durchschnittlich 5 bis 90 Mol% wiederkehrende Strukturelemente der Formel

(7) 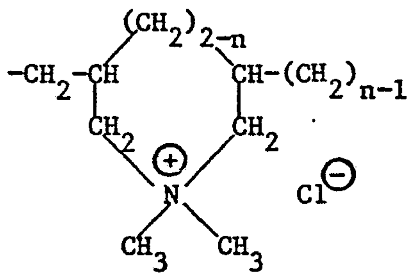

worin n 1 oder 2 ist, und durchschnittlich 1 bis 80 Mol% wiederkehrende Strukturelemente der Formel

(8) 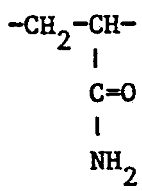

aufweisen.

Copolymerisate mit wiederkehrenden Strukturelementen der Formeln (7) und (8) sind z.B. in DE-A-3 029 306 beschrieben.

Bevorzugte Copolymerisate von Verbindungen der Acrylsäurereihe und quaternären Ammoniumsalzen der Acrylsäurereihe als Komponente (A) weisen ein Molekulargewicht von $10^4$ bis $5x10^6$ und in beliebiger Reihenfolge durchschnittlich 1 bis 5 Mol% wiederkehrende Strukturelemente der Formel

(9) 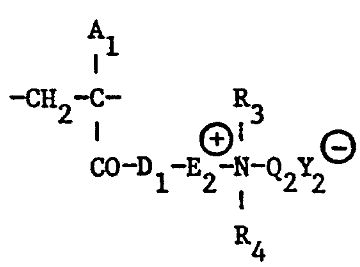 ,

durchschnittlich 75 bis 99 Mol% wiederkehrende Strukturelemente der Formel

(10) 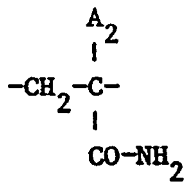

und 0 bis durchschnittlich 10 Mol% wiederkehrende Strukturelemente der Formeln

(11) 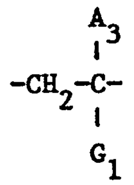 und gegebenenfalls

(12) 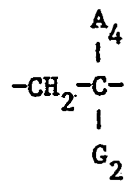

auf, worin $A_1$, $A_2$, $A_3$ und $A_4$ je Wasserstoff oder Methyl, $G_1$ und $G_2$ voneinander verschieden sind und je -CN, -COOH oder

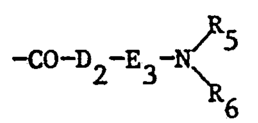 ,

$D_1$ und $D_2$ je Sauerstoff oder -NH-, $E_2$ und $E_3$ je Alkylen mit 1 bis 4 Kohlenstoffatomen, das gegebenenfalls durch Hydroxyl substituiert ist, $R_3$, $R_4$, $R_5$ und $R_6$ je Methyl oder Aethyl, $Q_2$ Alkyl, Hydroxyalkyl mit 1 bis 4 Kohlenstoffatomen oder Benzyl und $Y_2$ ein Halogenid-, Alkylsulfat- oder Alkylphosphonatanion mit 1 bis 4 Kohlenstoffatomen im Alkylrest bedeuten.

Als bevorzugte Bedeutungen für die Symbole der Formel (9) gelten für $A_1$ Methyl, für $D_1$ Sauerstoff, für $E_2$ unsubstituiertes n-Propylen oder insbesondere unsubstituiertes Aethylen, für $R_3$ und $R_4$ Methyl und für $Q_2$ unsubstituiertes Propyl, vorzugsweise Aethyl oder insbesondere Methyl. $A_2$ in Formel (10) steht vorzugsweise für Wasserstoff. Falls Strukturelemente der Formel (11) für sich allein, d.h. in Abwesenheit von Strukturelementen der Formel (12) vorhanden sind, gelten als bevorzugte Bedeutungen für die Symbole in Formel (11):

Methyl für $A_3$ und

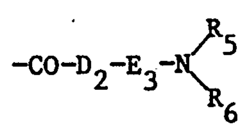

für $G_1$, wobei $D_2$, $E_3$, $R_5$ und $R_6$ die gleichen bevorzugten Bedeutungen haben wie für $D_1$, $E_2$, $R_3$ und $R_4$ vorstehend angegeben. Falls Strukturelemente der Formel (12) zusätzlich zu den Strukturelementen der Formel (II) vorhanden sind, bedeuten in Formel (12) $A_4$ vorzugsweise Wasserstoff und $G_2$ vorzugsweise -CN oder $A_4$ insbesondere Methyl und $G_2$ insbesondere -COOH.

Copolymerisate mit wiederkehrenden Strukturelementen der Formeln (9) und (10) sind z.B. in US-A-3996 146 beschrieben.

Als spezifische Beispiele der Komponente (B) der erfindungsgemässen Zusammensetzungen seien die folgenden Ammoniumsalze genannt:

(13) 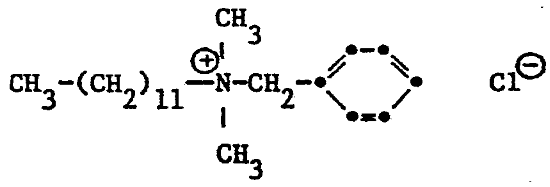

(14) 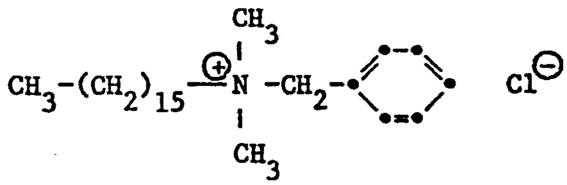

(15) 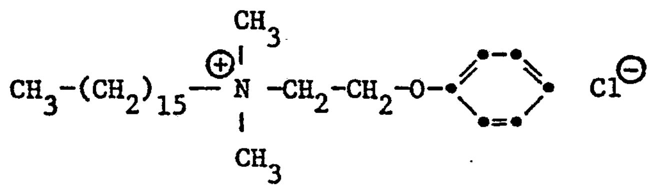

(16) 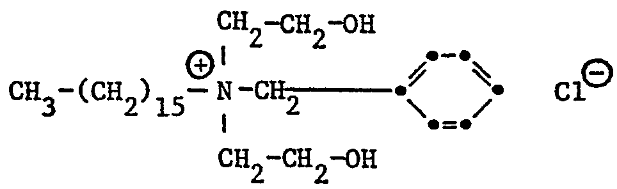

(17) 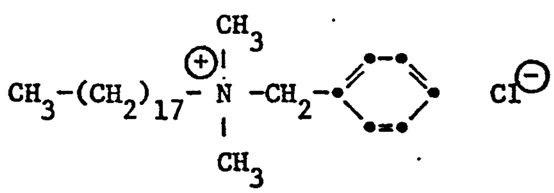

(18) 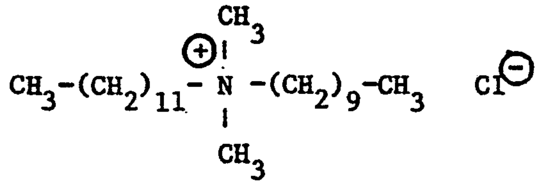

(19) 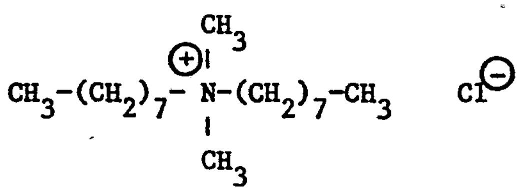

(20) 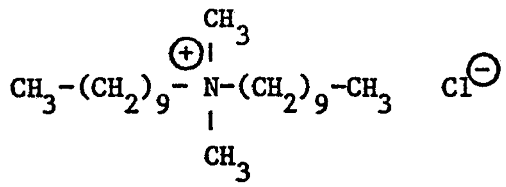

(21) 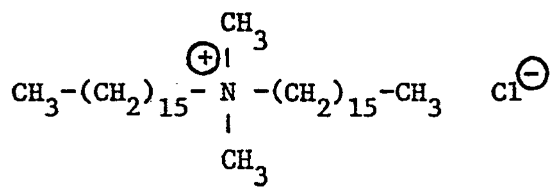

(22) 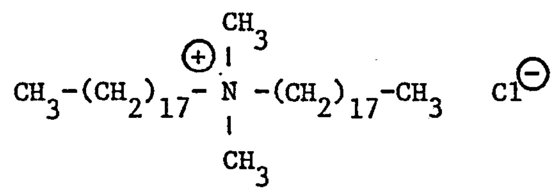

Hierbei stehen die Ammoniumsalze der Formel (13) und vor allem der Formeln (18) bis (22) im Vordergrund des Interesses.

In Formel (2) der anionischen Tenside, die als Komponente (C) in den erfindungsgemässen Zusammensetzungen enthalten sind, bedeutet $Z^{\oplus}$ insbesondere ein Natrium- oder ein Ammoniumkation, wobei das Ammoniumkation ($NH_4^{\oplus}$ unsubstituiert oder vorzugsweise durch 1, 2 oder vor allem 3 Alkyl- oder Alkanolreste mit je 1 bis 4 Kohlenstoffatomen substituiert ist.

Wie bereits angedeutet bedeutet $X^{\ominus}$ in Formel (2) den Rest oberflächenaktiver Sarkosinate, Sulfate, z.B. Alkylsulfate, Alkyläthersulfate, Alkylamidsulfate, Alkylamidäthersulfate, Alkylarylpolyäthersulfate oder Monoglyceridsulfate, Sulfonate, z.B. Alkylsulfonate, Alkylamidsulfonate, Alkylarylsulfonate, α-Olefinsulfonate oder Sulfobernsteinsäurederivate, z.B. Alkylsulfosuccinate, Alkyläthersulfosuccinate, Alkylamidsulfosuccinate, Alkylamidpolyäthersulfosuccinate oder Alkylsulfosuccinamide.

In Frage kommen z.B. für Sarkosinat-Tenside solche der Formel

(23) 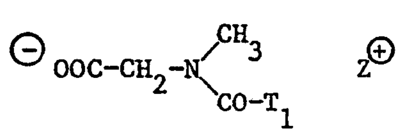 ,

worin $T_1$ Alkyl oder Alkenyl mit 7 bis 21, vorzugsweise 11 bis 17 Kohlenstoffatomen und $Z^{\oplus}$ die angegebenen Bedeutungen hat.

In Formel (23) leiten sich die Reste $T_1CO$- von den entsprechemden gesättigten oder ungesättigten Fettsäuren mit 8 bis 22, vorzugsweise 12 bis 18 Kohlenstoffatomen ab. Als Beispiele solcher Fettsäuren seien Capryl-, Caprin-, Arachin- und Behensäure, insbesondere Laurin-, Myristin-, Palmitin- und Stearinsäure oder Myristolein-, Palmitolein-, Elaeostearin-, Clupanodonsäure, insbesondere Oel-, Elaidin-, Eruka-, Linol- und Linolensäure genannt. Alkyl- und Alkenylreste für $T_1$, die sich von technischen Gemischen der genannten gesättigten und/oder ungesättigten Fettsäuren ableiten, sind besonders bevorzugt. Als spezifische Vertreter von Sarkosimat-Tensiden seien vor allem solche der Formeln

(24) 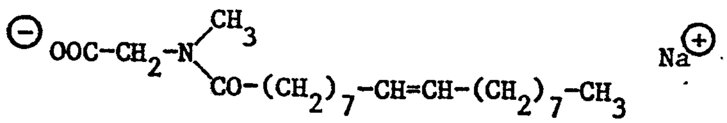

und insbesondere

(25) 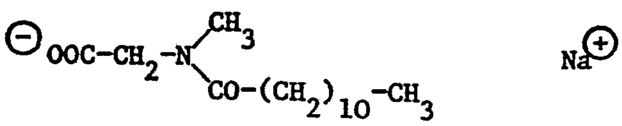

genannt.

Falls $X^{\ominus}$ einen Sulfatrest (von Alkylsulfaten, sowie von Aether-, Ester-, Amid- oder Aminosulfaten) bedeutet, kommen z.B. insbesondere Tenside der Formeln

(26) $T_1-O-(CH_2-CH_2-O)_{p-1}-SO_3^{\ominus}Z^{\oplus}$ ,

(27) 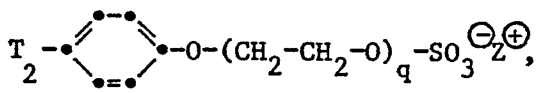

(28) $T_1-CO-NH-(CH_2)_r-O-SO_3^{\ominus}Z^{\oplus}$ oder

(29) $T_1-CO-NH-(CH_2-CH_2-O)_p-SO_3^{\ominus}Z^{\oplus}$

in Betracht, worin $T_2$ Alkyl oder Alkenyl mit 6 bis 14, vorzugsweise 8 bis 12 Kohlenstoffatomen, p eine ganze Zahl von 1 bis 50, q eine ganze Zahl von 6 bis 12 und r eine ganze Zahl von 2 bis 6 bedeuten und $T_1$ und $Z^{\oplus}$ die angegebenen Bedeutungen haben.

Bevorzugte Alkylreste für $T_2$ in Formel (27) sind z.B. Alkylreste mit 6 bis 12 Kohlenstoffatomen wie n-Hexyl, i-Hexyl, n-Heptyl, n-Octyl, i-Octyl, n-Nonyl, i-Nonyl, n-Decyl, n-Dodecyl, ferner Alkylreste mit 12-14 Kohlenstoffatomen, wie Lauryl und Myristyl und Alkenylreste, wie Oleyl und Reste, die sich von dimerisierten Olefinen mit je 6 oder 7 Kohlenstoffatomen ableiten.

Als spezifische Vertreter von Sulfat-Tensiden seien vor allem solche der Formeln

(30) $^{\ominus}O_3S-O-(CH_2)_{2-4}-NH-CO-(CH_2)_{11}-CH_3\ Na^{\oplus}$,

(31) $^{\ominus}O_3S-(O-CH_2-CH_2)_{8-10}-NH-CO-(CH_2)_{11}-CH_3\ Na^{\oplus}$ ,

(32) 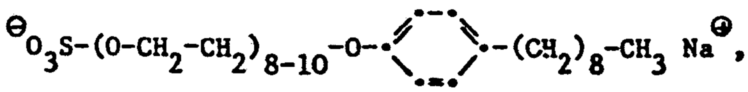

insbesondere

(33) $^{\ominus}O_3S-(O-CH_2-CH_2)_2-O-(CH_2)_{11}-CH_3\ Na^{\oplus}$ und

(34) 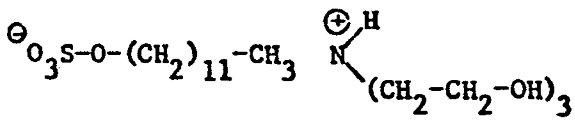

genannt.

Ist $X^{\ominus}$ ein Sulfonatrest, so kommen Tenside der Formeln

(35) 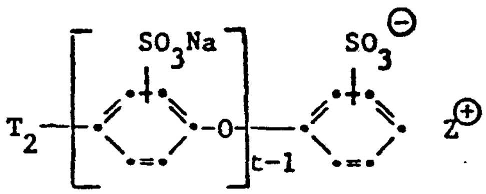 ,

(36) 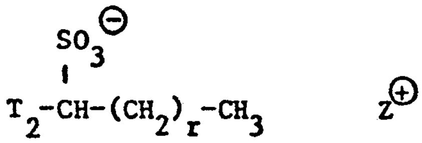 ,

(37) $T_2-COO-CH_2-CH_2-SO_3^{\ominus}\ Z^{\oplus}$ ,

(38) 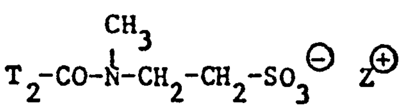 ,

(39) 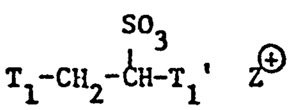 ,

(40) $T_1-CH=CH-SO_3^{\ominus}\ Z^{\oplus}$ oder

(41) 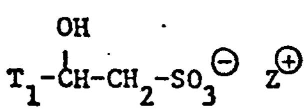

in Betracht, worin $T_1$, $T_2$, $Z^{\oplus}$ und r die angegebenen Bedeutungen haben, $T_1'$ die für $T_1$ angegebenen Bedeutungen hat, wobei $T_1$ und $T'_1$ gleich oder voneinander verschieden sind und t 1 oder 2 bedeutet.

Als spezifische Vertreter solcher Sulfonat-Tenside seien vor allem die Reste der Formeln

(42) $CH_3-(CH_2)_{11}-CH(SO_3^{\ominus})-(CH_2)_{2-4}-CH_3 \quad Na^{\oplus}$ ,

(43) $^{\ominus}O_3S-(CH_2)_2-OOC-(CH_2)_{10}-CH_3 \quad NH_4^{\oplus}$ ,

(44) 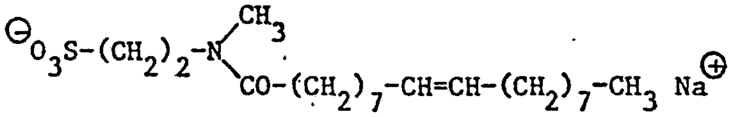 ,

insbesondere

(45) 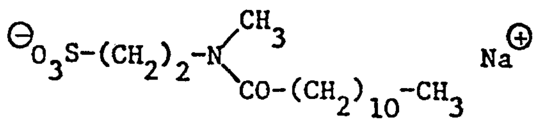 und

(46) 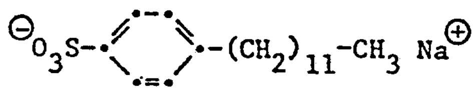

erwähnt.

Bedeutet $X^{\ominus}$ den Rest eines Sulfobernsteinsäurederivats, so entspricht das Tensid z.B. einer der Formeln

(47) $T_2-OOC-CH(SO_3^{\ominus})-CH_2-COO-[T_2']_{t-1}-[Na]_{2-t} \quad Z^{\oplus}$ ,

(48) $T_1-O-(CH_2-CH_2-O)_{p-1}-OC-CH_2-CH(SO_3^{\ominus})-COONa \quad Z^{\oplus}$ ,

(49) $T_1-CO-NH-(CH_2-CH_2-O)_{p-1}OC-CH_2-CH(SO_3^{\ominus})-COONa \quad Z^{\oplus}$ ,

(50) $T_1-OOC-CH_2-CH(SO_3^{\ominus})-COONa \quad Z^{\oplus}$ ,

(51) $T_2-NH-CO-CH_2-CH(SO_3^{\ominus})-COONa \quad Z^{\oplus}$ ,

(52) 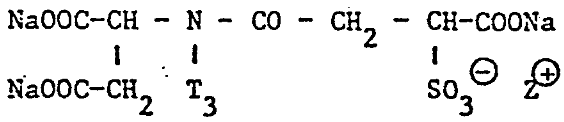 oder

(53) 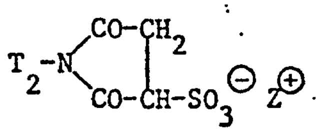

worin $T_1$, $T_2$, $Z^{\oplus}$, p und t die angegebenen Bedeutungen haben und $T_2'$ die für $T_2$ angegebenen Bedeutungen hat, wobei $T_2$ und $T'_2$ gleich oder voneinander verschieden sind.

Als spezifische Vertreter solcher Tenside seien die Sulfobernsteinsäurederivate der Formeln

(54) 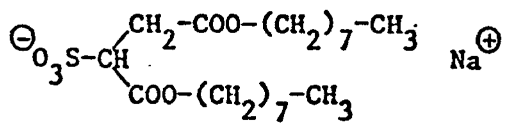 ,

(55) 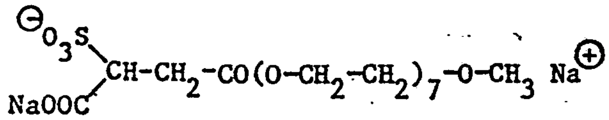 ,

(56) 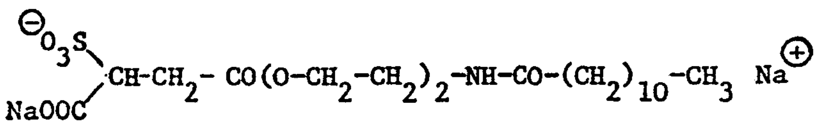 ,

(57) 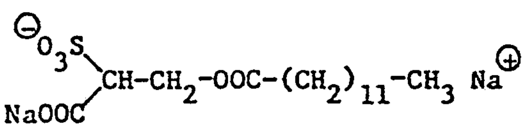 ,

(58) 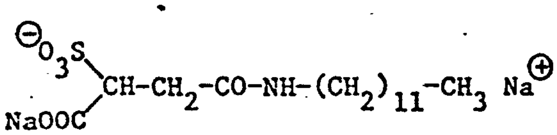 ,

(59) 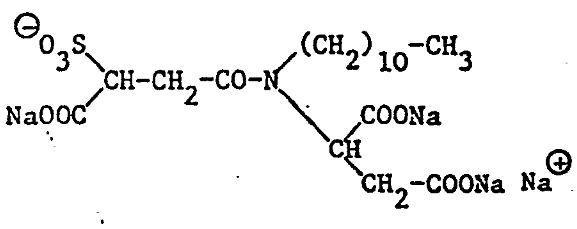 ,

(60) 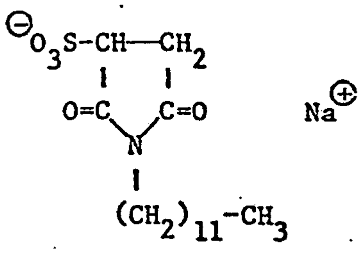 und insbesondere

(61) $$\begin{matrix} ^{\ominus}O_3S \\ NaOOC \end{matrix}\!\!>CH-CH_2-CO-(O-CH_2-CH_2-)_2-O-(CH_2)_{11}-CH_3 \quad Na^{\oplus}$$

erwähnt.

Im Vordergrund des Interesses stehen als Tenside für die Komponente (C) der erfindungsgemässen Zusammensetzungen oberflächenaktive Sarkosinate, Sulfate, Sulfonate und Sulfobernsteinsäurederivate der Formeln (25), (32), (34), (36), (45), (46) und (61).

Zur Herstellung der erfindungsgemässen Zusammensetzung wird z.B. so verfahren, dass man die Komponente (A) mit der Komponente (B) und mit der Komponente (C) in wässrigem Medium bei 10 bis 90°C, vorzugsweise 10 bis 60°C, insbesondere 15 bis 40°C, und einem pH-Wert von 3 bis 8 in der Regel während 30 bis 100, vorzugsweise 60 bis 90 Minuten vermischt, wobei man gleichzeitig das anionische Tensid als Komponente (C) mit dem polymeren Ammoniumsalz als Komponente (A) und mit dem monomeren Ammoniumsalz als Komponente (B) unter Ionenaustausch mindestens teilweise umsetzt, und stets ein Ueberschuss mindestens eines anionischen Tensids der Formel (2), bezogen auf das polymere Ammoniumsalz und auf das monomere, quaternäre Ammoniumsalz einsetzt.

Bei ihrer Verwendung in der Kosmetikindustrie werden die erfindungsgemässen Zusammensetzungen der Komponenten (A), (B) und (C) vorzugsweise als Haarkosmetika eingesetzt.

Die erfindungsgemässen kosmetischen Mittel, vorzugsweise haarkosmetische Mittel, liegen in ihrer bevorzugten Ausführungsform als wässrige Lösungen vor, die vorzugsweise 0,2 bis 1,0 Gewichtsteile, berechnet als Wirksubstanz, mindestens eines polymeren, quaternären Ammoniumsalzes als Komponente (A), vorzugsweise 0,2 bis 6 Gewichtsteile, berechnet als Wirksubstanz, mindestens einem monomeren quaternären Ammoniumsalzes als Komponente (B) und vorzugsweise 8 bis 15, insbesondere 9 bis 12 Gewichtsteile, berechnet als Wirksubstanz, mindestens eines anionischen Tensids als Komponente (C) und gegebenenfalls kosmetische Hilfsmittel als Komponente (D) enthalten und mit entionisiertem Wasser auf insgesamt 100 Gewichtsteile verdünnt sind.

Die fakultativen, kosmetischen Hilfsmittel als Komponente (D) sind handelsübliche Mittel, wie sie in der Haarkosmetik eingesetzt werden. In Betracht kommen z.B. Tenside, die von den als Komponente (C) eingesetzten Tensiden der angegebenen Art verschieden sind, wie Polyglycerolester und Polyglykolester von Fettsäuren, insbesondere Polyglyceroloeate, im weiteren auch Polyglykole und Schaumstabilisatoren, z.B. Fettsäurepolyalkanolamide, Verdickungsmittel natürlicher oder synthetischer Herkunft wie Hydroxypropylmethylcellulose und Polyacrylsäure, Opalisierungsmittel, wie Fettsäuremonoalkanol-amide oder vorzugsweise Glycerinmonostearat, Hautschutzmittel wie Eiweisshydrolysate und Allantoine ferner auch u.a. Konservierungsmittel, Parfüms und Perlglanzmittel.

Erforderlichenfalls werden die haarkosmetischen Mittel auf einen pH-Wert von 3 bis 8, vorzugsweise 5 bis 8 eingestellt, was zweckmässig durch einen Zusatz von wässrigen Lösungen aus z.B. Natriumhydroxid oder vorzugsweise Zitronensäure erreicht werden kann.

Bei der Applikation der haarkosmetischen Mittel, vorzugsweise auf menschlichem Haar, wird im Haarbehandlungsverfahren das vorstehend beschriebene, wässrige, haarkosmetische Mittel auf mit Leitungswasser angefeuchtetem Haar, in der Regel bei Raumtemperatur bis leicht erhöhter Temperatur, z.B. 20 bis 40°C, aufgebracht und das Haar anschliessend shampooniert und gleichzeitig konditioniert. Das so behandelte Haar kann ebenfalls in Form von Perücken oder Toupets vorliegen.

Der wesentliche Vorteil der vorliegenden Erfindung liegt darin, dass bei der Applikation der haarkosmetischen Mittel, welche in neuartiger Kombination sowohl die polymeren als auch die monomeren, quaternären Ammoniumsalze als Komponenten (A) und (B) neben den Tensiden der angegebenen Art als Komponente (C) enthalten, nicht nur eine gute Reinigung, sondern auch gleichzeitig ausgezeichnete Konditioniereffekte auf dem behandelten Haar erzielt werden. So weisen die mit der erfindungsgemässen Zusammensetzung behandelten Haare antistatische Eigenschaften und eine sehr gute Kämmbarkeit (nass und trocken) auf. Zudem ist bei wiederholter Verwendung der erfindungsgemässen Zusammensetzung keinerlei unerwünschter Akkumuliereffekt unter Beeinträchtigung des Griffs, der Fülle und des Glanzes der behandelten Haare zu beobachten. Im Vergleich zu den Konditioniereffekten auf Haaren, die mit herkömmlichen kosmetischen Mitteln behandelt werden, weisen die mit den erfindungsgemässen haarkosmetischen Mitteln behandelten Haare somit in unerwarteter Weise ausserordentlich gute Konditioniereffekte auf. Die üblichen kosmetischen Mittel enthalten zwar auch Mischungen aus Tensiden und quaternären Ammoniumsalzen, jedoch nicht die neuartige und erfinderische Kombination von ausgewählten polymeren, von ausgewählten monomeren, quaternären Ammoniumsalzen und von ausgewählten Tensiden gemäss der vorliegenden Erfindung. Als weiteren Vorteil der erfindungsgemässen Zusammensetzung sei erwähnt, dass Konditioniereffekte verleihende Zusammensetzungen mit den ausgewählten anionischen Tensiden der angegebenen Art ohne Zusatz von relativ teuren zwitterionischen oder amphoteren Tensiden erzielt werden können. Dementsprechend sind die erfindungsgemässen Zusammemsetzungen besonders preisgünstig.

In den nachfolgenden Beispielen angegebene Teile und Prozente sind Gewichtsprozente.

**Beispiel I**

Lösung A: 100 Teile des anionischen Tensides der Formel (34) werden mit 400 Teilen entionisiertem Wasser bei 25°C zu 500 Teilen Tensidlösung verdünnt.

Lösung B: 5 Teile eines im Handel erhältlichen, quaternären, polymeren Cellulose-Derivates, dessen Celluloseeinheiten Reste der Formel (3) enthalten (POLYMER®JR 400), werden bei 20°C in kleinen Portionen innerhalb von 30 Minuten in 100 Teilen entionisierten Wasser eingetragen. Es entsteht eine viskose Lösung.

Lösung C: Die Lösung B des quaternären, polymeren Ammoniumsalzes (105 Teile) wird nun in 350 Teilen der Tensidlösung A innerhalb von 80 Minuten bei 35°C zugegeben, wobei gleichzeitig die Umsetzung der quaternären Strukturelemente des polymeren Celluloseäther-Derivates mit dem eingesetzten, anionischen Tensids unter Ionenaustausch erfolgt. Man erhält 455 Teile einer wässrigen, schwachopalisierenden und viskosen Lösung.

Suspension D: In die verbleibende Portion (150 Teile) der Tensidlösung B werden 10 Teile des monomeren, quaternären Ammoniumsalzes der Formel (22) bei 80°C innerhalb von 30 Minuten unter Rühren eingetragen, wobei gleichzeitig die Umsetzung des quaternären Ammoniumsalzes mit dem eingesetzten anionischen Tensid unter Ionenaustausch erfolgt. Man erhält 160 Teile einer feinen Suspension, die auf 20°C abgekühlt wird.

Erfindungsgemässe Zusammensetzung: Die Suspension D wird nun unter Rühren bei 20°C innerhalb von 15 Minuten in die Lösung C eingetragen. Man erhält 615 Teile einer wässrigen, trüben Lösung, die durch Zugabe einer 5%igen, wässrigen Natriumhydroxydlösung auf den PH-Wert von 7,1 eingestellt und mit entionisiertem Wasser auf 1000 Teile verdünnt wird.

Applikation der Zusammensetzung: Nun wird die verdünnte trübe Lösung auf eine mit Leitungswasser angefeuchtete Perücke aus ungebleichtem und ungefärbtem, braunem Menschenhaar europäischer Herkunft bei 40°C in dreimaliger Applikation à 2 Shampoonierungen im sogenannten Halbseitentest aufgebracht, worauf das Perückenhaar bei dieser Temperatur shampooniert und gleichzeitig konditioniert wird. Im Halbseitentest wird nur die eine Hälfte der Perücke mit der vorstehend genannten Lösung shampooniert und konditioniert, während die andere Hälfte der Perücke mit einer Lösung unter gleichen Bedingungen shampooniert wird, die kein erfindungsgemässes Gemisch aus den Ammoniumsalzen und dem Tensid, sondern nur das Tensid enthält. In dieser sogenannten Leerformulierung wird die Menge an Ammoniumsalzen durch die entsprechende Menge Tensid ersetzt, so dass z.B. die Leerformulierung als Vergleich 11,5% des Tensides der Formel (34) enthält, wobei der pH-Wert der Leerformulierung ebenfalls mit der wässrigen, 5%igen Natriumhydroxydlösung auf den pH-Wert von 7,1 eingestellt wird. Nach jeder Applikation wird die Nass- und Trockenkämmbarkeit der erfindungsgemäss behandelten Perückenhälfte im Vergleich zu der mit der Leerformulierung behandelten Perückenhälfte mit Hilfe der nachfolgenden Notenskala beurteilt.

+3 viel besser als die Leerformulierung
+2 besser als die Leerformulierung
+1 etwas besser als die Leerformulierung
0 kein Unterschied zur Leerformulierung
-1 etwas schlechter als die Leerformulierung
-2 schlechter als die Leerformulierung
-3 viel schlechter als die Leerformulierung.

Die erzielten Kämmbarkeitsnoten sind in der nachfolgenden Tabelle I zusammengefasst.

**Tabelle 1**

| | nach der 1. Applikation | nach der 2. Applikation | nach der 3. Applikation |
|---|---|---|---|
| Nasskämmbarkeit | 0 | +1 | +1 |
| Trockenkämmbarkeit | +(1-2) | +1 | +(1-2) |

Zudem weist die erfindungsgemäss behandelte Perückenhälfte bessere antistatische Eigenschaften auf als die mit der Leerformulierung behandelte Perückenhälfte. Die antistatischen Effekte werden qualitativ erfasst, indem die Neigung der Haare zum "Wegfliegen" (fly away) subjektiv beurteilt wird. Auf beiden Perückenhälften können keinerlei Akkumuliereffekte nach 3 Applikationen beobachtet werden.

Zu Vergleichszwecken wird eine analoge Zusammensetzung hergestellt, die jedoch kein monomeres, quaternäres Ammoniumsalz der Formel (22) enthält, sondern lediglich 10 Teile des polymeren, quaternären Celluloseäther-Derivates und 100 Teile des anionischen Tensides der Formel (34) enthält. Im Gegensatz zur

erfindungsgemässen Zusammensetzung erhält man nach der 1., 2. und 3. Applikation der bekannten Zusammensetzung ohne monomeres, quaternäres Ammoniumsalz auf der Perückenhälfte jeweils die Note Null bis -(0-1) im Vergleich zur Leerformulierung für die Nass- und Trockenkämmbarkeit. Zudem werden keine guten antistatischen Eigenschaften der mit der bekannten Zusammensetzung behandelten Haare erzielt.

**Beispiel 2**

Man verfährt wie im Beispiel 1 angegeben, setzt jedoch 5 Teile eines im Handel erhältlichen, quaternären, polymeren Guargummi-Derivates, dessen Polygalaktomannan-Einheiten Reste der Formel (1) enthalten (JAGUAR ® C 13 S), 10 Teile des monomeren Ammoniumsalzes der Formel (22) und 100 Teile des anionischen Tensides der Formel (34) ein.

Man erhält 615 Teile einer trüben Lösung, die durch Zugabe einer 5%igen, wässrigen Natriumhydroxydlösung auf den pH-Wert von 7,1 eingestellt und mit entionisiertem Wasser auf 1000 Teile verdünnt wird.

Das Shampoonieren und Konditionieren der Perücke im Halbseitentest mit obiger Lösung wird ebenfalls wie in Beispiel 1 durchgeführt, wobei die folgenden, in der nachstehenden Tabelle 11 angegebenen Kämmbarkeitsnoten erzielt werden:

**Tabelle II**

| | nach der 1. Applikation | nach der 2. Applikation | nach der 3. Applikation |
|---|---|---|---|
| Nasskämmbarkeit | +(2-3) | +3 | +3 |
| Trockenkämmbarkeit | +(2-3) | +3 | +3 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

Zu Vergleichszwecken wird eine analoge Zusammensetzung hergestellt, die jedoch kein monomeres, quaternäres Ammoniumsalz der Formel (22) enthält, sondern lediglich 10 Teile des polymeren, quaternären Guargummi-Derivates und 100 Teile des anionischen Tensides der Formel (34). Im Gegensatz zur erfindungsgemässen Zusammensetzung betragen die Nasskämmbarkeitsnoten jeweils +2 nach der 1. und 2. Applikation und +(2-3) nach der 3. Applikation auf der Perückenhälfte der bekannten Zusammensetzung, die kein monomeres Ammoniumsalz enthält.

Aehnliche Ergebnisse werden bein Ersatz des polymeren, quaternären Guargummi-Derivates mit Gruppen der Formeln (3) bis (6), beim Ersatz des monomeren, quaternären Ammoniunsalzes der Formel (22) durch diejenigen einer der Formeln (13) bis (21), und beim Ersatz des anionischen Tensids der Formel (34) durch diejenigen einer der Formeln (25), (42), (45), (46) oder (61) erzielt.

**Beispiel 3**

Man verfährt wie in Beispiel 1 angegeben, setzt jedoch als polymeres, quaternäres Ammoniumsalz 3 Teile des in Beispiel 2 angegebenen Guargummi-Derivates, 10 Teile des monomeren quaternären Ammoniumsalzes der Formel (22) und 100 Teile des anionischen Tensides der Formel (34) ein.

Man erhält 613 Teile einer trüben Lösung, die durch Zugabe einer 5%igen, wässrigen Natriumhydroxyd-Lösung auf den pH-Wert von 7,1 eingestellt und mit entionisiertem Wasser auf 1000 Teile verdünnt wird.

Das Shampoonieren und Konditionieren der Perücke im Halbseitentest mit obiger Lösung wird ebenfalls wie in Beispiel 1 durchgeführt, wobei die folgenden, in der nachstehenden TabelleIII angegebenen Kämmbarkeitsnoten erzielt werden:

**Tabelle III**

| | nach der 1. Applikation | nach der 2. Applikation | nach der 3. Applikation |
|---|---|---|---|
| Nasskämmbarkeit | +(2-3) | +3 | +3 |
| Trockenkämmbarkeit | +(2-3) | +3 | +3 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

**Beispiel 4**

Man verfährt wie in Beispiel 1 angegeben, setzt jedoch als polymeres, quaternäres Ammoniumsalz 3 Teile des in Beispiel angegebenen Guargummi-Derivates, 10 Teile des monomeren quaternären Ammoniumsalzes der Formel (20) und 100 Teile des anionischen Tensides der Formel (34) ein.

Man erhält 613 Teile einer schwach opalisierenden Lösung, die durch Zugabe einer 5%igen, wässrigen Natriumhydroxyd-Lösung auf den pH-Wert von 7,1 eingestellt und mit entionisiertem Wasser auf 1000 Teile verdünnt wird.

Das Shampoonieren und Konditionieren der Perücke im Halbseitentest mit obiger Lösung wird ebenfalls wie in Beispiel 1 durchgeführt, wobei die folgenden, in der nachstehenden Tabelle IV angegebenen Kämmbarkeitsnoten erzielt werden:

**Tabelle IV**

| | nach der 1. Applikation | nach der 2. Applikation | nach der 3. Applikation |
|---|---|---|---|
| Nasskämmbarkeit | +3 | +3 | +3 |
| Trockenkämmbarkeit | +(1-2) | +(1-2) | +(1-2) |

Die erfindungsgemäss behandelte Perückenhälfte im Vergleich zur Perückenhälfte, die mit der Leerfornulierung behandelt wird, weist ebenfalls gute antistatische Eigenschaften auf, wobei auf beiden Perückenhälften keinerlei Akkumuliereffekte nach 3 Applikationen beobachtet werden können.

Gleiche Ergebnisse, insbesondere gleiche Kämmbarkeitsnoten werden erzielt, wenn man an Stelle von 3 Teilen des in Beispiel 2 angegebenen, im Handel erhältlichen Guargumni-Derivates (JAGUAR® C 13 S), ein anderes, im Handel erhältlichen Guargummi-Derivat anderer Provenienz (COSMEDIA GUAR® C 261) einsetzt.

**Beispiel 5**

Man verfährt wie in Beispiel 1 angegeben, setzt jedoch als polymeres, quaternäres Ammoniumsalz 3 Teile des in Beispiel 2 angegebenen Guargummi-Derivates, 10 Teile des monomeren quaternären Ammoniumsalzes der Formel (22) und 100 Teile des anionischen Tensides der Formel (33) ein.

Man erhält 613 Teile einer trüben und viskosen Lösung, die durch Zugabe einer 10%igen, wässrigen Zitronensäure-Lösung auf den pH-Wert von 7,1 eingestellt und mit entionisiertem Wasser auf 1000 Teile verdünnt wird.

Das Shampoonieren und Konditionieren der Perücke im Halbseitentest mit obiger Lösung wird ebenfalls wie

in Beispiel 1 durchgeführt, wobei die folgenden, in der nachstehenden Tabelle V angegebenen Kämmbarkeitsnoten erzielt werden:

**Tabelle V**

| | nach der 1. Applikation | nach der 2. Applikation | nach der 3. Applikation |
|---|---|---|---|
| Nasskämmbarkeit | +2 | +(2-3) | +(2-3) |
| Trockenkämmbarkeit | +2 | +2 | +2 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

**Beispiel 6**

Man verfährt wie in Beispiel 1 angegeben, setzt jedoch als polymeres, quaternäres Ammoniumsalz 3 Teile des in Beispiel 2 angegebenen Guargummi-Derivates, 10 Teile des monomeren quaternären Ammoniumsalzes der Formel (20) und 100 Teile des anionischen Tensides der Formel (33) ein.

Man erhält 613 Teile einer opalisierenden bis leicht trüben und viskosen Lösung, die durch Zugabe einer 10%igen, wässrigen Zitronensäure-Lösung auf den pH-Wert von 7,1 eingestellt und mit entionisiertem Wasser auf 1000 Teile verdünnt wird.

Das Shampoonieren und Konditionieren der Perücke im Halbseitentest mit obiger Lösung wird ebenfalls wie in Beispiel 1 durchgeführt, wobei die folgenden, in der nachstehenden Tabelle VI angegebenen Kämmbarkeitsnoten erzielt werden:

**Tabelle VI**

| | nach der 1. Applikation | nach der 2. Applikation | nach der 3. Applikation |
|---|---|---|---|
| Nasskämmbarkeit | +3 | +3 | +3 |
| Trockenkämmbarkeit | +(1-2) | +(1-2) | +(1-2) |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

**Beispiel 7**

Man verfährt wie in Beispiel 1 angegeben, setzt jedoch als polymeres, quaternäres Ammoniunsalz 5 Teile eines im Handel erhältlichen Copolymerisates,das Strukturelemente der Formeln (7) und (8) und ein Molekulargewicht von etwa $2x10^6$ aufweist (MERQUAT ® 550), 10 Teile des monomeren, quaternären Ammoniumsalzes der Formel (20) und 100 Teile des anionischen Tensides der Formel (34) ein.

Man erhält 615 Teile einer opalisierenden bis leicht trüben Lösung, die durch Zugabe einer 5%igen, wässrigen Natriumhydroxyd-Lösung auf den den pH-Wert von 7,1 eingestellt und mit entionisiertem Wasser auf 1000 Teile verdünnt wird.

Das Shampoonieren und Konditionieren der Perücke im Halbseitentest mit obiger Lösung wird ebenfalls wie in Beispiel 1 durchgeführt, wobei die folgenden, in der nachstehenden Tabelle VII angegebenen Kämmbarkeitsnoten erzielt werden:

**Tabelle VII**

| | nach der 1. Applikation | nach der 2. Applikation | nach der 3. Applikation |
|---|---|---|---|
| Nasskämmbarkeit | +(1-2) | +(1-2) | +(1-2) |
| Trockenkämmbarkeit | 0 | +1 | 0 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

**Beispiel 8**

Man verfährt wie in Beispiel 1 angegeben, setzt jedoch als polymeres, quaternäres Ammoniunsalz 4 Teile eines im Handel erhältlichen Copolymerisates, das Strukturelemente der Formeln (9) und (10) aufweist (RETEN ® 210), 10 Teile des monomeren, quaternären Ammoniumsalzes der Formel (22) und 100 Teile des anionischen Tensides der Formel (34) ein.

Man erhält 613 Teile einer trüben Lösung, die durch Zugabe einer 5%igen, wässrigen Natriumhydroxyd-Lösung auf den pH-Wert von 7,1 eingestellt und mit entionisiertem Wasser auf 1000 Teile verdünnt wird.

Das Shampoonieren und Konditionieren der Perücke im Halbseitentest mit obiger Lösung wird ebenfalls wie in Beispiel 1 durchgeführt, wobei die folgenden, in der nachstehenden Tabelle VIII angegebenen Kämmbarkeitsnoten erzielt werden:

**Tabelle VIII**

| | nach der 1. Applikation | nach der 2. Applikation | nach der 3. Applikation |
|---|---|---|---|
| Nasskämmbarkeit | +2 | +(2-3) | +(2-3) |
| Trockenkämmbarkeit | +3 | +3 | +3 |

Die erfindungsgemäss behandelte Perückenhälfte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

**Beispiel 9**

Man verfährt wie in Beispiel 1 angegeben, setzt jedoch als polymeres, quaternäres Ammoniumsalz 3 Teile des in Beispiel 2 angegebenen Guargummi-Derivates, 10 Teile des monomeren quaternären Ammoniumsalzes der Formel (20) und 150 Teile des anionischen Tensides der Formel (34) ein.

Man erhält 663 Teile einer trüben Lösung, die durch Zugabe einer 10 %igen, wässrigen Zitronensäure-Lösung auf den pH-Wert von 6,0 eingestellt und mit entionisiertem Wasser auf 1000 Teile verdünnt wird.

Das Shampoonieren und Konditionieren der Perücke im Halbseitentest mit der verdünnten Lösung wird ebenfalls wie in Beispiel 1 durchgeführt, wobei die folgenden, in der nachstehenden Tabelle IX angegebenen Kämmbarkeitsnoten erzielt werden:

**Tabelle IX**

| | nach der 1. Applikation | nach der 2. Applikation | nach der 3. Applikation |
|---|---|---|---|
| Nasskämmbarkeit | + (2-3) | + 3 | + 3 |
| Trockenkämmbarkeit | + 1 | + (1-2) | + (1-2) |

Die erfindungsgemäss behandelte Perückenhälte im Vergleich zur Perückenhälfte, die mit der Leerformulierung behandelt wird, weist ebenfalls gute antistatische Eigenschaften auf, wobei auf beiden Perückenhälften keinerlei Akkumuliereffekte nach 3 Applikationen beobachtet werden können.

**Patentansprüche**

1. Wässrige Zusammensetzungen aus polymeren, quaternären Ammoniumsalzen, aus monomeren, quaternären Ammoniumsalzen und aus anionischen, sulfonierten Tensiden oder Sarkosinattensiden, dadurch gekennzeichnet, dass sie einen pH-Wert von 3 bis 8 aufweisen und

(A) 0,05 bis 1,5 Gewichtsteile, berechnet als Wirksubstanz eines in wässrigen Tensidsystemen löslichen oder mikroemulgierbaren, Polymeren Ammoniumsalzes mit mindestens 6 quaternären Stickstoffatomen und einem Molekulargewicht von 10 bis $5x10^6$, das quaternierte Stärke-, Cellulose- oder Guargummi-Derivate, Copolymerisate aus Dialkenyldialkylammoniunsalzen und Acylamid oder Methacrylamid oder Copolymerisate aus Acryl- oder Methacrylsäure, -amid oder -nitril und quaternierten Ammoniumsalzen der Acrylreihe enthält,

(B) 0,2 bis 10 Gewichtsteile, berechnet als Wirksubstanz, eines Ammoniumsalzes der Formel

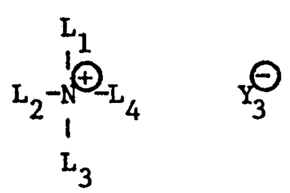 ,

worin $L_1$ und $L_2$ je Methyl, Aethyl oder Hydroxyäthyl, $L_3$ Alkyl mit 10 bis 22 Kohlenstoffatomen, $L_4$ Alkyl mit 10 bis 22 Kohlenstoffatomen, Benzyl, Phenoxymethylen oder Phenoxyäthylen und $Y_3^{\ominus}$ ein Chlorid-, Methylsulfat- oder Acetatanion bedeuten und

(C) 5 bis 20 Gewichtsteile, berechnet als Wirksubstanz, eines anionischen Tensids der Formel

$$X^{\ominus}Z^{\oplus} ,$$

worin $X^{\ominus}$ den Rest eines oberflächenaktiven Sarkosinates, Sulfats (Sulfatäthers), Sulfonates oder Sulfobernsteinsäurederivates und $Z^{\oplus}$ ein Alkalimetall- oder ein gegebenenfalls durch ($C_1$-$C_4$)-Alkyl- oder -Alkanolreste substituiertes Ammoniumkation bedeuten,

enthalten,
wobei das anionische Tensid mindestens teilweise mit den Komponenten (A) und (B) unter Ionenaustausch umgesetzt ist und die Zusammensetzungen mit entionisiertem Wasser auf insgesamt 100 Gewichtsteile verdünnt sind.

2. Zusammensetzungen nach Anspruch 1, dadurch gekennzeichnet, dass sie als Komponente (A) quaternäre Stärke-, Cellulose- oder Guargummi-Derivate enthalten, die ein Molekulargewicht von $10^3$ bis $10^6$ und Stärke-, Cellulose- oder Polygalaktomannan-Einheiten aufweisen, die mit 1-Trimethylammonium-2-hydroxypropyl-Rest mindestens teilweise veräthert sind, wobei die Stärke-, Cellulose- oder Polygalaktomannan-Einheiten Reste der Formeln

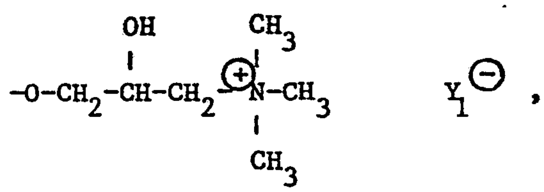

worin $Y_1^{\ominus}$ ein Halogenid-, Alkylsulfat- oder Alkylphosphonatanion oder das Anion einer Alkylcarbonsäure mit je 1 bis 4 Kohlenstoffatomen im Alkylrest bedeutet, und gegebenenfalls Reste der Formeln

$-O-(CH_2-CH_2-O)_{m_1}-H$,

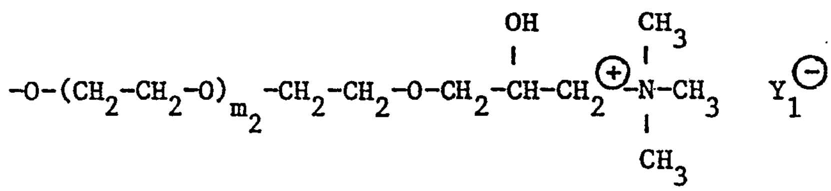

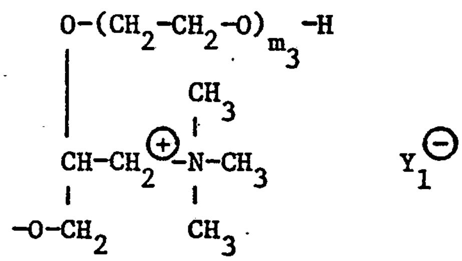

enthalten,

worin $m_1$, $m_2$ und $m_3$ jeweils ganze Zahlen von 1 bis 50 bedeuten und $Y_1^{\ominus}$ die angegebene Bedeutung hat.

3. Zusammensetzungen nach Anspruch 1, dadurch gekennzeichnet, dass sie als Komponente (A) ein Copolymerisat aus Dimethyldiallylammoniumchlorid und Acrylamid, das ein Molekulargewicht von $5x10^3$ bis $5x10^6$ und in beliebiger Reihenfolge durchschnittlich 5 bis 90 Mol% wiederkehrende Strukturelemente der Formel

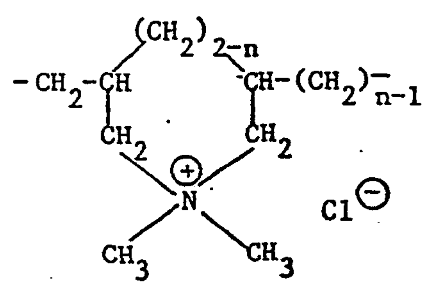

worin n 1 oder 2 ist, und durchschnittlich 1 bis 80 Mol % wiederkehrende Struktureelemente der Formel

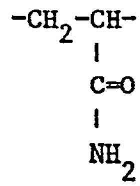

aufweist oder ein Copolymerisat aus Verbindungen der Acrylsäurereihe und quaternäre Ammoniumsalzen der Acrylsäurereihe, welches ein Molekulargewicht von $10^4$ bis $5x10^6$ und in beliebiger Reihenfolge durchschnittlich 1 bis 5 Mol % wiederkehrende Strukturelemente der Formel

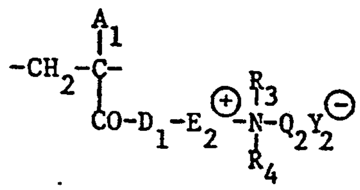 ,

durchschnittlich 75 bis 99 Mol % wiederkehrende Strukturelemente der Formel

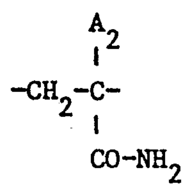 und

0 bis durchschnittlich 10 Mol % wiederkehrende Strukturelemente der Formel

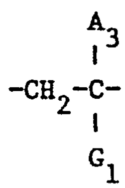 und gegebenenfalls 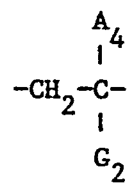

aufweisen, worin $A_1$, $A_2$, $A_3$ und $A_4$ je Wasserstoff oder Methyl, $G_1$ und $G_2$ voneinander verschieden sind und je -CN, -COOH oder

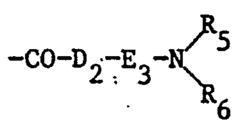 ,

$D_1$ und $D_2$ je Sauerstoff oder -NH-, $E_2$ und $E_3$ je Alkylen mit 1 bis 4 Kohlenstoffatomen, das gegebenenfalls durch Hydroxyl substituiert ist, $R_3$, $R_4$, $R_5$ und $R_6$ je Methyl oder Aethyl, $Q_2$ Alkyl, Hydroxyalkyl mit 1 bis 4 Kohlenstoffatomen oder Benzyl und $Y_2\ominus$ ein Halogenid-, Alkylsulfat- oder Alkylphosphonatanion mit 1 bis 4 Kohlenstoffatomen im Alkylrest bedeuten, enthalten.

4. Zusammensetzung nach Anspruch 1, dadurch gekennzeichnet, dass sie als Komponente (C) ein Tensid einer der Formeln

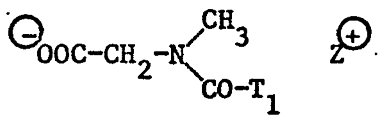

$T_1-O-(CH_2-CH_2-O)_{p-1}-SO_3^{\ominus}Z^{\oplus}$ ,

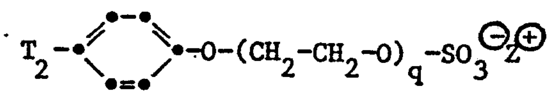 ,

$T_1-CO-NH-(CH_2)_r-O-SO_3^{\ominus}Z^{\oplus}$ ,

$T_1-CO-NH-(CH_2-CH_2-O)_p-SO_3^{\ominus}Z^{\oplus}$ ,

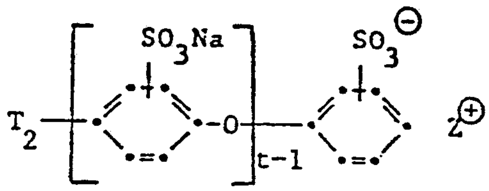 ,

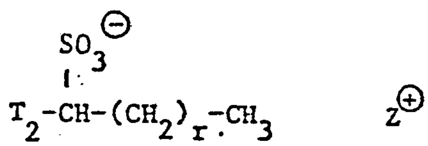 ,

$T_2-COO-CH_2-CH_2-SO_3^{\ominus}\ Z^{\oplus}$ ,

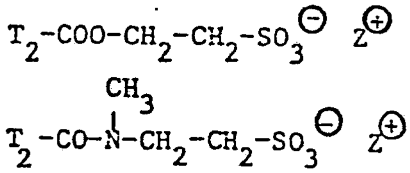 ,

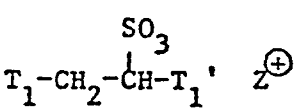 ,

$T_1-CH=CH-SO_3^{\ominus}\ Z^{\oplus}$ ,

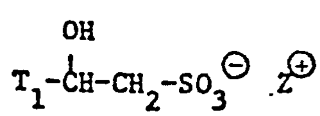 ,

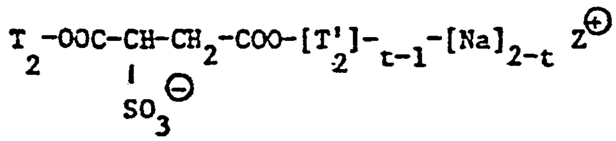,

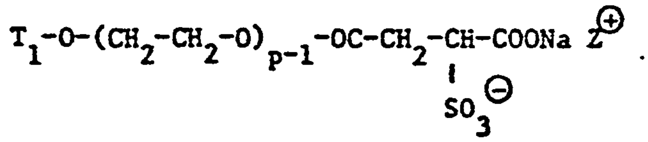,

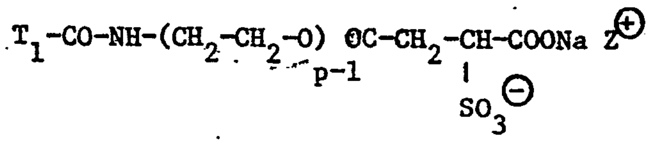,

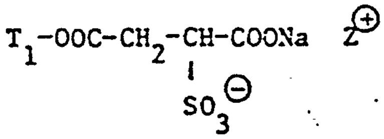,

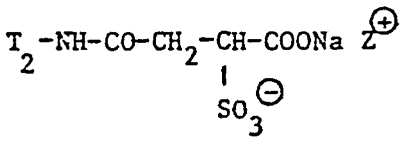,

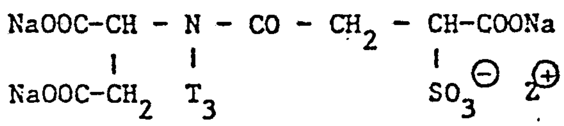 oder

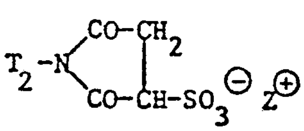

enthalten, worin $T_1$ und $T'_1$ je Alkyl oder Alkenyl mit 7 bis 21 Kohlenstoffatomen, $T_2$ und $T'_2$ je Alkyl oder Alkenyl mit 6 bis 14 Kohlenstoffatomen, p eine ganze Zahl von 1 bis 50, q eine ganze Zahl von 6 bis 12, r eine ganze Zahl von 1 bis 50, q eine ganze Zahl von 6 bis 12, r eine ganze Zahl von 2 bis 6 und t 1 oder 2 bedeuten und $Z^{\oplus}$ die in Anspruch 1 angegebenen Bedeutungen hat.

5. Verfahren zur Herstellung der Zusammensetzung gemäss einem der Ansprüche 1 bis 4, dadurch gekennzeichnet, dass man die Komponente (A) mit der Komponente (B) und mit der Komponente (C) in wässrigem Medium bei 10 bis 90°C und einem pH-Wert von 3 bis 8 vermischt, wobei man gleichzeitig das anionische Tensid als Komponente (C) mit dem polymeren Ammoniumsalz als Komponente (A) und mit dem monomeren Ammoniumsalz als Komponente (B) unter Ionenaustausch mindestens teilweise umsetzt.

6. Verwendung der Zusammensetzung gemäss einem der Ansprüche 1 bis 4 in haarkosmetischen Mitteln.

**Claims**

1. An aqueous composition of a polymeric quaternary ammonium salt, a monomeric, quaternary ammonium salt and an anionic, sulfonated surfactant or sarcosinate surfactant, which composition has a pH value of 3 to 8 and contains

(A) 0.05 to 1.5 parts by weight, calculated as active ingredient, of a polymeric ammonium salt which is soluble or microemulsifiable in aqueous surfactant systems and which has at least 6 quaternary nitrogen atoms

and a molecular weight of $10^3$ to $5x10^6$, which polymeric ammonium salt contains quaternised starch, cellulose or guar gum derivatives, copolymers formed from dialkenyldialkylammonium salts and acrylamide or methacrylamide or copolymers formed from acrylic acid, acrylamide, acrylonitrile, methacrylic acid, methacrylamide or methacrylonitrile and quaternary ammonium salts of the acrylic acid series,

(B) 0.2 to 10 parts by weight, calculated as active ingredient, of an ammonium salt of the formula

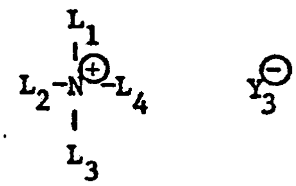 ,

wherein each of $L_1$ and $L_2$ is methyl, ethyl or hydroxyethyl, $L_3$ is $C_{10}$-$C_{22}$alkyl, $L_4$ is $C_{10}$-$C_{22}$alkyl, benzyl, phenoxymethylene or phenoxyethylene and $Y_3^{\ominus}$ is a chloride, methylsulfate or acetate anion and

(C) 5 to 20 parts by weight, calculated as active ingredient, of an anionic surfactant of the formula

$X^{\ominus}Z^{\oplus}$

wherein $X^{\ominus}$ is the radical of a surface-active sarcosinate, sulfate (sulfate ether), sulfonate or sulfosuccinic acid derivative and $Z^{\oplus}$ is an alkali metal cation or an ammonium cation which is unsubstituted or substituted by $C_1$-$C_4$alkyl or $C_1$-$C_4$alkanol radicals,

which anionic surfactant is at least partially reacted with the components (A) and (B) under ion exchange conditions and which composition is diluted with deionised water to a total of 100 parts by weight.

2. A composition according to claim 1, which contains, as the component (A), quaternary starch, cellulose or guar gum derivatives which have a molecular weight of $10^3$ to $10^6$ and contain starch, cellulose or polygalactomannan units which are at least partially etherified with the 1-trimethyl-ammonium-2-hydroxypropyl radical, which starch, cellulose or polygalactomannan units contain radicals of the formula

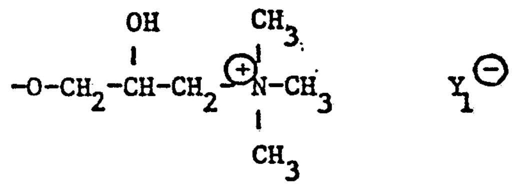

wherein $Y_1^{\ominus}$ is a halide, alkylsulfate or alkylphosphonate anion or the anion of an alkylcarboxylic acid, each alkyl radical containing 1 to 4 carbon atoms, and, if appropriate, radicals of the formulae

$$-O-(CH_2-CH_2-O)_{m_1}-H,$$

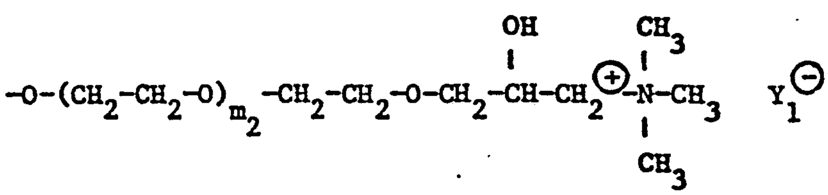

and

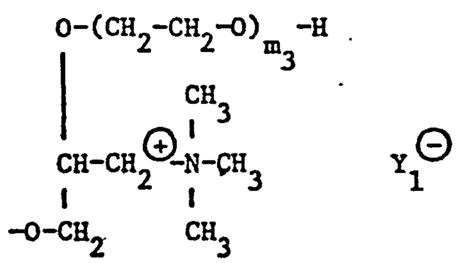

in which formulae each of $m_1$, $m_2$ and $m_3$ is an integer from 1 to 50 and $Y_1^{\ominus}$ is as defined.

3. A composition according to claim 1, which contains, as the component (A), a copypolymer of dimethyldiallylammonium chloride and acrylamide, which copolymer has a molecular weight of $5x10^3$ to $5x10^6$

and contains, in any disired sequence, an average of 5 to 90 mol% of recurring structural elements of the formula

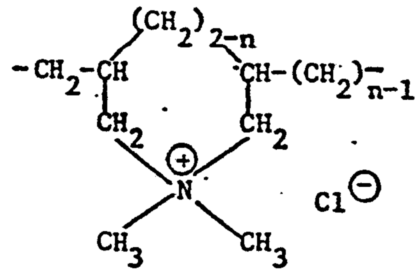

wherein n is 1 or 2, and an average of 1 to 80 mol% of recurring structural elements of the formula

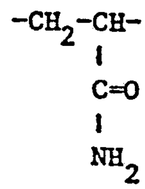

or a copolymer of compunds if the acrylic acid series and quaternary ammonium salts of the acrylic acid series, which copolymer has a molecular weight of $10^4$ to $5x10^6$ and contains, in any desired sequence, an average of 1 to 5 mol% of recurring structural elements of the formula

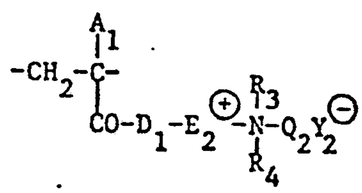

an average of 75 to 99 mol% of recurring structural elements of the formula

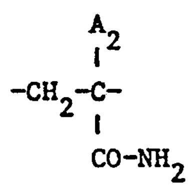

and 0 to an average of 10 mol% of recurring structural elements of the formula

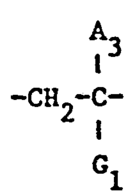 and, if appropriate, 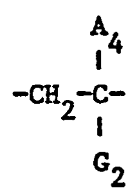

in which formulae each of $A_1$, $A_2$, $A_3$ and $A_4$ is hydrogen or methyl, $G_1$ and $G_2$ are different from one another and are each

-CN, -COOH or 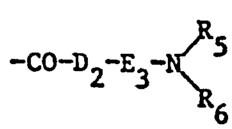 ,

each of $D_1$ and $D_2$ is oxygen or -NH-, each of $E_2$ and $E_3$ is $C_1$-$C_4$alkylene which is unsubstituted or substituted by hydroxy, each of $R_3$, $R_4$, $R_5$ and $R_6$ is methyl or ethyl, $Q_2$ is alkyl, $C_1$-$C_4$hydroxyalkyl or benzyl and $Y_2^{\ominus}$ is a halide, alkylsulfate or alkylphosphonate anion having 1 to 4 carbon atoms in the alkyl radical.

4. A composition according to claim 1, which contains, as the component (C), a surfactant of one of the formulae

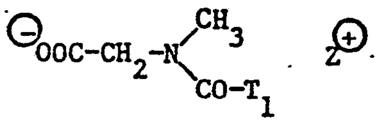

$T_1-O-(CH_2-CH_2-O)_{p-1}-SO_3^{\ominus}Z^{\oplus}$ ,

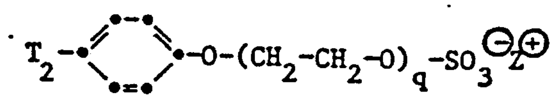 ,

$T_1-CO-NH-(CH_2)_r-O-SO_3^{\ominus}Z^{\oplus}$ ,

$T_1-CO-NH-(CH_2-CH_2-O)_p-SO_3^{\ominus}Z^{\oplus}$ ,

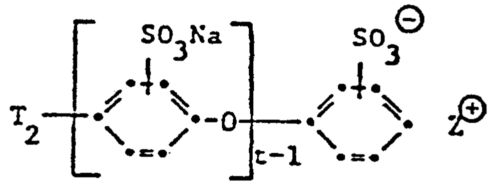 ,

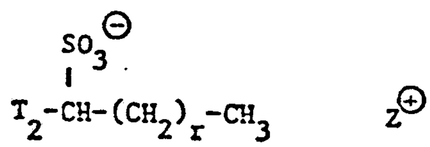 ,

$T_2-COO-CH_2-CH_2-SO_3^{\ominus}\ Z^{\oplus}$ ,

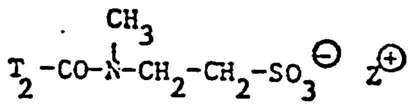 ,

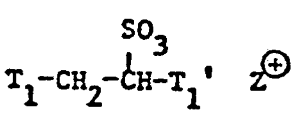 ,

$T_1-CH=CH-SO_3^{\ominus}\ Z^{\oplus}$ ,

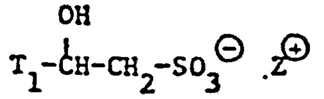 ,

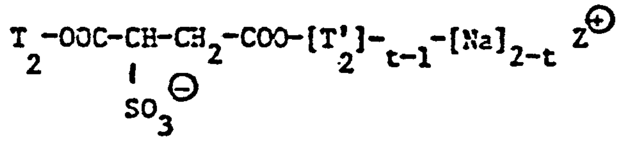 ,

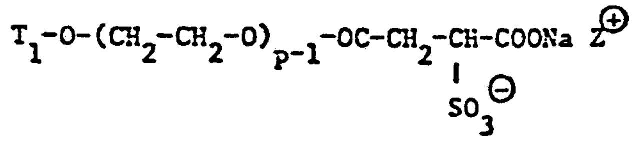 ,

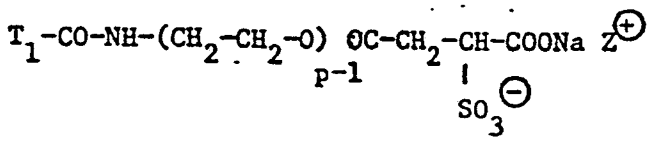 ,

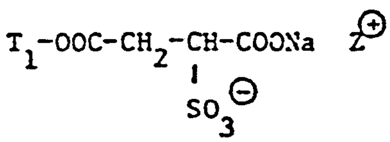 ,

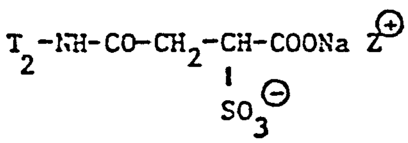 ,

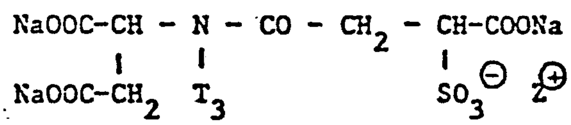 or

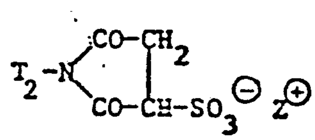 .

in which formulae each of $T_1$ and $T'_1$ is $C_7$-$C_{21}$alkyl or $C_7$-$C_{21}$alkenyl, each of $T_2$ and $T'_2$ is $C_6$-$C_{14}$alkyl or $C_6$-$C_{14}$-alkenyl, p is an integer from 1 to 50, q is an integer from 6 to 12, r is an integer from 2 to 6 and t is 1 or 2 and $Z^{\oplus}$ is as defined in claim 1.

5. A process for the preparation of a composition according to any one of claims 1 to 4, which process comprises mixing, in an aqueous medium at 10 to 90°C and at a pH value of 3 to 8, the component (A) with the component (B) and with the component (C), the anionic surfactant, as the component (C), being simultaneously at least partially reacted with the polymeric ammonium salt, as the component (A), and with the monomeric ammonium salt, as the component (B), under ion exchange conditions.

6. Use of a composition according to any one of claims 1 to 4 in cosmetic compositions for the hair.

**Revendications**

1. Compositions aqueuses à base de sels polymères d'ammonium quaternaire, de sels monomères d'ammonium quaternaire et de tensioactifs sulfonés anioniques ou de tensioactifs sarcosinates, caractérisées en ce qu'elles ont un pH de 3 à 8 et qu'elles contiennent

(A) 0,05 à 1,5 partie en poids, calculée en tant que matière active, d'un sel d'ammonium polymère, soluble ou microémulsionnable dans des systèmes tensioactifs aqueux, ayant au moins 6 atomes d'azote quaternaire et une masse moléculaire de $10^3$ à $5x10^6$, et contenant des dérivés quaternisés de l'amidon, de la cellulose ou de la gomme de guar, des copolymères de sels de dialcényldialkylammonium et d'acrylamide ou de méthacrylamide, ou des copolymères de l'acide acrylique ou de l'acide méthacrylique, de l'acrylamide ou du méthacrylamide, ou de l'acrylonitrile ou du méthacrylonitrile, et de sels d'ammonium quaternaire de la série acrylique,

(B) 0,2 à 10 parties en poids, calculées en tant que matière active, d'un sel d'ammonium de formule

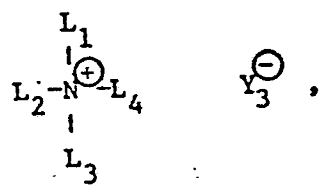 ,

dans laquelle $L_1$ et $L_2$ sont chacun le radical méthyle, éthyle ou hydroxyéthyle, $L_3$ est un radical alkyle ayant de 10 à 20 atomes de carbone, $L_4$ est un radical alkyle ayant de 10 à 22 atomes de carbone, ou le radical benzyle, phénoxyméthylène ou phénoxyéthylène, et $Y_3^\ominus$ est un anion chlorure, méthylsulfate ou acétate, et

(C) 5 à 20 parties en poids, calculées en tant que matière active, d'un tensioactif anionique de formule

$X^\ominus Z^\oplus$

dans laquelle $X^\ominus$ est le résidu d'un sarcosinate, d'un sulfate (éther-sulfate) d'un sulfonate ou d'un dérivé de l'acide sulfosuccinique tensioactifs, et $Z^\oplus$ est un cation/métal alcalin, ou un cation ammonium éventuellement substitué par des radicaux alkyle ou hydroxyalkyle en $C_1$-$C_4$, où on fait réagir au moins partiellement, par échange d'ions, le tensioactif anionique sur les constituants (A) et (B), et on dilue les compositions à un total de 100 parties en poids avec de l'eau désionisée.

2. Compositions selon la revendication 1, caractérisées en ce qu'elles contiennent en tant que constituant (A) des dérivés quaternaires de l'amidon, de la cellulose ou de la gomme de guar, qui présentent une masse moléculaire de $10^3$ à $10^6$ et des motifs amidon, cellulose ou polygalactomannane qui sont au moins partiellement éthérifiés par un radical 1-triméthylammonium-2-hydroxypropyle, les motifs amidon, cellulose ou polygalactomannane contenant des radicaux de formules

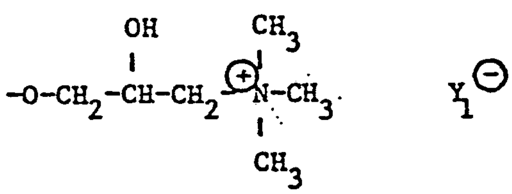 ,

où $Y_1^\ominus$ est un anion halogénure, alkylsulfate ou alkylphosphonate, ou l'anion d'un acide alkylcarboxylique ayant dans la partie alkyle 1 à 4 atomes de carbone, et éventuellement des radicaux de formules

$-O-(CH_2-CH_2-O)_{m_1}-H$,

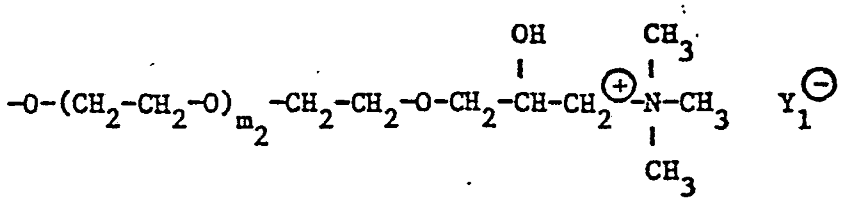

et

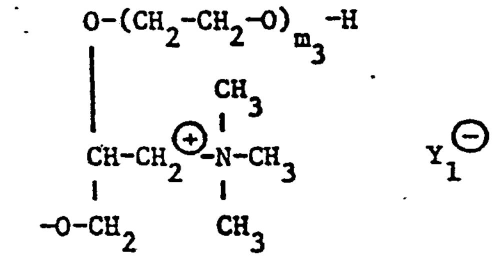

dans lesquelles $m_1$, $m_2$ et $m_3$ sont chacun des nombres entiers de 1 à 50, et $Y_1^{\ominus}$ a la signification donnée cidessus.

3. Compositions selon la revendication 1, caractérisées en ce qu'elles contiennent en tant que constituant (A) un copolymère de chlorure de diméthyldiallylammonium et d'acrylamide, qui a une masse moléculaire de $5x10^3$. à $5x10^6$ et, dans un ordre quelconque, en moyenne 5 à 90 % molaire d'éléments structuraux répétitifs de formule

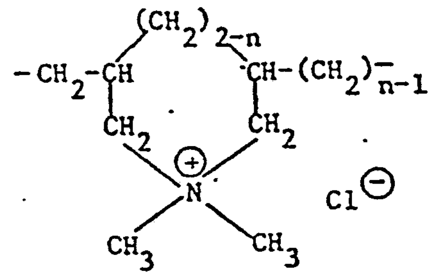

dans laquelle n vaut 1 ou 2, et en moyenne 1 à 80 % molaire d'éléments structuraux répétitifs de formule

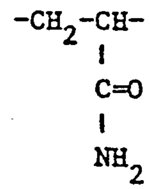

ou encore un copolymère de composés de la série de l'acide acrylique et de sels d'ammonium quaternaire de la série de l'acid acrylique, lequel polymère a une masse moléculaire de $10^4$ à $5x10^6$ et présente dans un ordre quelconque en moyenne 1 à 5 % molaire d'éléments structutaux répétitifs de formule

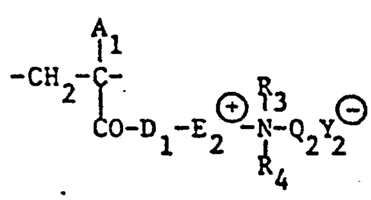

,

en moyenne de 75 à 99 % molaire d'éléments structuraux répétitifs de formule

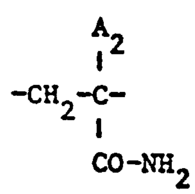

et de 0 à en moyenne 10 % molaire d'éléments structuraux répétitifs de formule

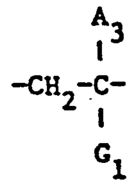

et éventuellement

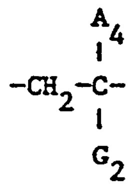

où $A_1$, $A_2$, $A_3$ et $A_4$ sont chacun l'hydrogène ou le radical méthyle, $G_1$ et $G_2$ sont différents l'un de l'autre et sont chacun le radical

-CN, -COOH ou 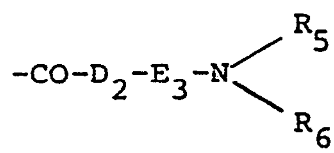 ,

$D_1$ et $D_2$
sont chacun l'oxygène ou le radical -NH-, $E_2$ et $E_3$ sont chacun un radical alkylène ayant de 1 à 4 atomes de carbone, éventuellement substitué par un radical hydroxyle, les radicaux $R_3$, $R_4$, $R_5$ et $R_6$ sont chacun le radical méthyle ou éthyle, $Q_2$ est un radical alkyle, hydroxylakyle ayant de 1 à 4 atomes de carbone ou le radical benzyle, et $Y_2^{\ominus}$ est un anion halogénure, alkylsulfate ou alkylphosphonate ayant de 1 à 4 atomes de carbone dans le fragment alkyle.

4. Composition selon la revendication 1, caractérisée en ce qu'elle contient en tant que constituant (C) un tensioactif ayant l'une des formules suivantes

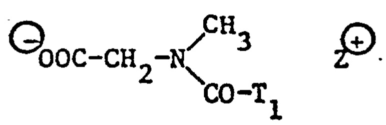

$$T_1-O-(CH_2-CH_2-O)_{p-1}-SO_3^{\ominus}Z^{\oplus} ,$$

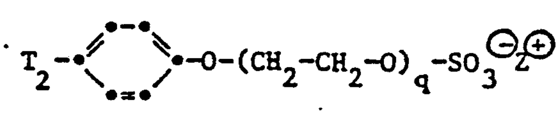 ,

$$T_1-CO-NH-(CH_2)_r-O-SO_3^{\ominus}Z^{\oplus} ,$$

$$T_1-CO-NH-(CH_2-CH_2-O)_p-SO_3^{\ominus}Z^{\oplus} ,$$

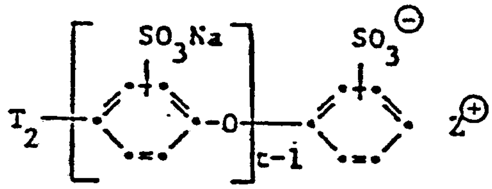 ,

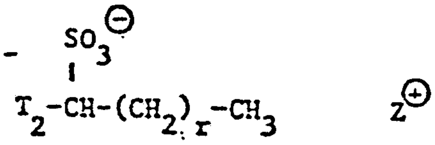 ,

$T_2-COO-CH_2-CH_2-SO_3^{\ominus}\ Z^{\oplus}$ ,

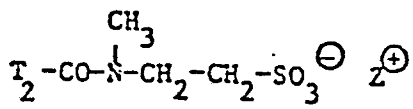 ,

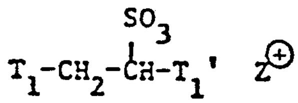 ,

$T_1-CH{=}CH-SO_3^{\ominus}\ Z^{\oplus}$ ,

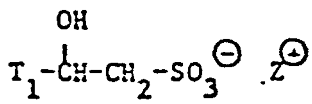 ,

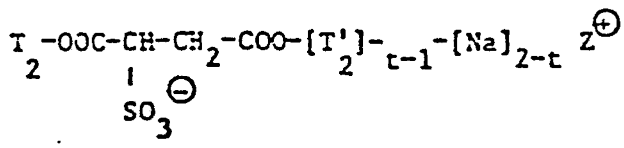 ,

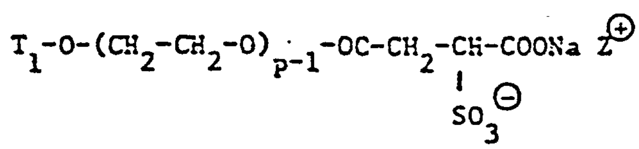 ,

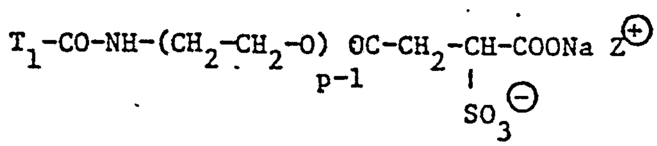 ,

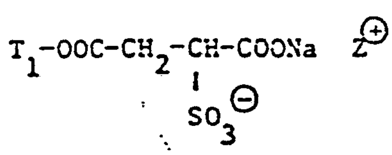 ,

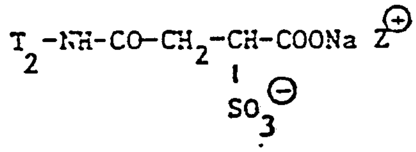 ,

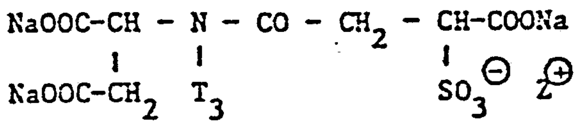 ou bien

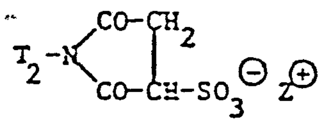 .

dans lesquelles $T_1$ et $T_1'$ sont chacun un radical alkyle ou alcényle ayant de 7 à 21 atomes de carbone, $T_2$ et $T_2'$ sont chacun un radical alkyle ou alcényle ayant de 6 à 14 atomes de carbone, p est un nombre entier de 1 à 50, q est un nombre entier de 6 à 12, r est un nombre entier de 2 à 6 et t vaut 1 ou 2, et $Z^{\ominus}$ a les significations données dans la revendication 1.

5. Procédé pour la préparation de la composition selon l'une des revendications 1 à 4, caractérisé en ce qu'on mélange le constituant (A) au constituant (B) et au constituant (C) en milieu aqueux à 10 - 90°C et à un pH de 3 à 8, tout en faisant réagir, au moins partiellement, et par échange d'ions, le tensioactif anionique en tant que constituant (C) sur le sel d'ammonium polymère en tant que constituant (A) et sur le sel d'ammonium monomère en tant que constituant (B).

6. Utilisation de la composition selon l'une des revendications 1 à 4 dans des produits cosmétiques pour cheveux.